# EUROPEAN PATENT SPECIFICATION

(11) **EP 2 838 482 B1**
(45) Date of publication and mention of the grant of the patent: **09.03.2016**
(21) Application number: 13718497.4
(22) Date of filing: 18.04.2013
(51) Int. Cl.: A61F 13/02, A61L 15/58, A61L 24/00

(54) **AN ADHESIVE WAFER**
HAFTSCHEIBE
PLAQUETTE ADHÉSIVE

(30) Priority: 20.04.2012 DK 201270203
(43) Date of publication of application: 25.02.2015
(73) Proprietor: Coloplast A/S, 3050 Humblebæk (DK)
(72) Inventor: OEELUND, Jakob, 3450 Alleroed (DK)
(86) International application number: PCT/DK2013/050110
(87) International publication number: WO 2013/156034

(56) References cited:
- WO-A1-99/40903
- US-A- 6 011 194
- US-A1- 2010 217 215

## Description

### Field of the invention

The present invention relates to an adhesive wafer, especially a soft wafer for application to the skin or a wound or for attaching a collecting device for collecting human waste to the skin.

### Background

Wound dressings comprising soft adhesive or very thin wound dressings may be difficult to handle during application as they may easily wrinkle or cling to themselves. The same situation may occur when handling ostomy appliances with soft adhesive base plates.

Whereas a conventional hydrocolloid adhesive wafer is rather stiff and thereby easy to handle and apply, the soft adhesive wafers are soft and mechanical unstable and may easily fold and stick to themselves during application.

When applying an adhesive wafer around a stoma or over a wound the conventional hydrocolloid adhesive wafers are relatively stable and easy to handle, even when the protection layer covering the skin-contacting adhesive surface before application is fully removed prior to application. The constructions of the current hydrocolloid adhesives are carried out in such a manner that the wafer, when the protection layer is removed, is stiff enough in order for the wafer to stay in an almost planar manner when held in horizontal position. In other words, the wafer does not bend, curl or fold significantly during application.

This is due to the choice of backing layer and adhesive. The backing layer is usually a relatively stiff polymer backing film with a high modulus of elasticity and the adhesive is a polymer based continuous phase filled with particles that add to the modulus of the adhesive. The combination of a high modulus backing and adhesive makes the adhesive wafer quite stiff.

Adhesive wafers made from soft adhesives are also known and facilitate enhanced flexibility and comfort to the user. However, when handling wafers made from soft adhesives, and combined with low modulus backing layers, problems may occur in the application situation. Such adhesive wafers are very soft, flexible and unable to hold themselves in a relatively planar manner after removal of the protection layer. The adhesive wafer itself is so flexible that the side portions of the wafer will bend down with gravity after removal of protection layer, resulting in the adhesive may stick to itself, bend, curl or fold and the wafer will be damaged even before it is applied to the body.

### 2. Description of the Related Art

Handling soft and/or thin adhesive wafers or dressings may be addressed in different ways. The adhesive surface may be covered with a number of protection layers and/or the backing layer may be provided with detachable support means.

WO99/40903 discloses transdermal delivery devices having a layer of adhesive, a backing layer and a release liner wherein at least one of the release liner and the backing layer is porous (e.g. paper). Suitable porous synthetic polymeric materials may be used. The use of a porous release liner and/or backing layer provides improved results in storage, i.e. less loss of the active material. Paper is preferred as porous material.

Wound dressings are often provided with two protection layers, each covering an area of the wafer. Hereby, the user can remove one protection layer, attach the exposed adhesive surface to the body and then remove the second protection layer and apply the remaining part of the dressing, all the way through without touching the adhesive surface of the dressing with the fingers. This solution is for hygiene purpose and is often referred to as a non-touch solution.

Ultra thin wound dressings may be provided with a support layer on the non-adhesive surface of the wafer. Such support layer may be in the form of a rigid or semi-rigid frame that controls the conformation of the dressing during application. After the dressing has been applied, the frame is removed.

Today, conventional ostomy appliances are typically provided with a single protection layer, covering the entire adhesive surface. A non-touch solution is achieved by having a non-adhesive tab or ear on the edge portion of the flange for holding during application without touching the adhesive. The tab or ear may also be used to ease detachment of the wafer later. This solution is suited for mechanical stable wafers while soft wafers comprising soft adhesive would be difficult - not to say impossible - to handle with such protection layer system.

Thus, there is still a need for an adhesive wafer having a high flexibility and comfort for the user and being easy to apply.

### Summary of the invention

In one aspect of the invention, it relates to an adhesive wafer comprising a protection layer for improving handling and application of soft adhesive wafers.

In one aspect of the invention, it relates to an adhesive wafer for an ostomy appliance with a protection layer that facilitates easy and stepwise application of the adhesive wafer.

In yet an aspect of the invention, it relates to an adhesive wafer that can easily be positioned over a wound or around a stoma, preferably without the need of repositioning and unintended contact with the adhesive surface.

### Brief description of the drawings

Figure 1 shows an embodiment of the invention shown from the skin facing side,
Figure 2 shows the same embodiment where a portion of the protection layer is removed,
Figure 3 shows another embodiment of the invention shown from the skin facing side,
Figure 4 shows the same embodiment where a portion of the protection layer is removed and
Figure 5 shows the same embodiment seen in cross-section.

### Detailed Disclosure

It is an object of the invention to provide an adhesive wafer suitable for attachment to the skin or a wound, the wafer comprising a dressing sheet comprising a layer of skin-friendly adhesive, the adhesive layer being provided with a backing layer on the non-skin-facing surface thereof, and a releasable flexible protection layer on the skin-facing surface of the adhesive layer, the protection layer protecting the adhesive layer before use of the wafer and the protection layer being attached to the adhesive layer in such a way that it is releasable and wherein the protection layer is in the form of a porous material being a foam and having a thickness of 500 µm - 3 mm.

By protection layer is meant a layer attached releasably to the adhesive surface of the dressing sheet. The protection layer is detachable and is intended for protecting the adhesive surface before application. The layer may also serve as an application tool for easing the application of the dressing sheet to the skin. When the dressing sheet is fully applied, the protection layer is absent from the dressing sheet and is thus not a part of the dressing sheet in use but merely present during storage and optionally during application. The protection layer may be provided with a non-stick surface in order to facilitate an easy release of the adhesive from the layer. Such non-stick surface may be achieved in the form of a coating on the surface of the protection layer or the layer may comprise a material with such non-stick properties towards the used adhesive.

The character of the non-stick surface depends on the composition of the adhesive.

By porous is meant a three-dimensional material comprising void volumes of air or gas. The material may be permeable to air or liquid or non permeable. The material is a foam, which may be open-celled or closed celled.

The permeability of the material is not crucial, as the protection layer is removed before or during application and is thus not present on the wafer during use.

Another way of expressing porosity is by the density of the material. The material may be comprised by a skeletal portion, often called the "matrix" or "frame" and a gas void portion. The density of a porous material will be lower than the density of the skeletal material in solid, compact form, the higher degree of gas voids, the lower is the density of the material.

Due to the porosity of the material, the protection layer may be rather thick compared to material conventionally used for such protection layers and may be perceived as being three-dimensional - having a perceivable thickness. Conventional protection layers of paper or polymer films typically have a thickness of 100-120 µm and may be perceived as a two-dimensional structure.

The protection layer of the adhesive wafer may have a substantially uniform thickness of 500 µm - 3 mm, such as 700 µm - 2.5 mm and even 1-2 mm.

The protection layer may be thicker than the dressing sheet. By dressing sheet is herein meant the backing layer with adhesive layer. The dressing sheet may further be provided with an absorbent layer. The thickness of the absorbent layer should be excluded when measuring the thickness of the dressing sheet in this comparison.

The porous material is light-weight, flexible, but appears preferably more rigid than the dressing sheet it carries due to the thickness of the protection layer. The incorporation of air volumes in the material at the same time provides stiffness and softness when the material is bended as well as it feels more pleasant than a thin, stiff protection layer. A protection layer made from a solid (not porous) material, such as a polymer film or paper, may act as a blade spring when bended, fiercely trying to return to its inherited flat configuration. A porous material, such as a foam layer may also try to return to the flat configuration, but more soft and slowly due to the gas voids acting as buffers/airbags. Furthermore, the porous material is pleasant to touch and handle compared to a conventional protection layer made solid polymer or paper, which may have sharp edges.

Soft adhesive wafers, such as wound dressings and ostomy appliances may be difficult to handle in the application situation, especially when the protection layer is removed, as they require a solution for proper handling during application of the wafer to the skin, because the handling of such is very difficult as it will bend and is not able to stay in a planar configuration without support. The wafer of the present invention provides a solution where the porous structure of the protection layer provides handling properties allowing the user to easily apply the dressing sheet to the skin preventing wrinkling or adhering of the dressing sheet to itself.

In a preferred embodiment, the protection layer is in the form of two or more separately removable portions. By dividing the protection layer into two or more separately removable portions, the first portion of the protection layer may be removed and the exposed adhesive surface is applied to the body, thereby bonding the adhesive dressing sheet partially to the skin. Then the second and optionally the third portion of the protection layer are removed in order to attach the remaining adhesive surface of the dressing sheet to the skin.

The separately removable portions of the protection layer may be arranged symmetrically over the adhesive surface or they may be asymmetrical.

The presence of two or more separately removable portions of the protection layer may render it possible to apply the wafer without touching the adhesive surface.

The wafer of the present invention may preferably be provided with a layer of a soft absorbent adhesive. Examples of such adhesives may be a polyalkyleneoxide polymer and an organosiloxane based cross-linked adhesive system.

In a preferred embodiment of the invention, the adhesive comprises ethylene vinyl acetate. The adhesive comprising ethylene vinyl acetate may suitably be an adhesive known in the art such as the adhesive composition disclosed, for example in International Patent Application 2009/006901.

Other suitable adhesives for the wafer may be silicone or polyurethane based adhesives.

The adhesive may be directly attached to the backing layer or it may be in the form of a laminate. Such laminate may comprise a reinforcing layer and/or it may comprise a second adhesive layer.

The adhesive layer may cover the entire skin-facing surface of the wafer or it may be partly absent, either in a pattern of multiple small holes or in the form of one or more larger perforations, for example at the central portion of the wafer.

The adhesive wafer may comprise a thin elastic, low modulus backing layer covered with a soft absorbing adhesive on one surface. The adhesive layer may be in the form of one or more layers.

The porous material is preferably made from a polymer material. The polymer may be foamed into the porous structure or it may be processed into fibrous, porous material.

The porous material may be in the form of a foam. The foam material may be a polyolefin. Preferred polyolefines may be polyethylene (PE) or polypropylene (PP).

The protection layer may be in the form of a single protection layer covering the entire adhesive surface or it may be in the form of two or more portions, together covering the entire adhesive surface of the dressing sheet. The protection layer portions may be overlapping on at least a part of the adhesive or they may be adjoined along a line or curve.

The protection layer being divided into at least two portions facilitates that the portions may be removed one by one during application, for example removing a first portion of the protection layer, attaching the thereby exposed adhesive surface to the skin, then removing the next portion attaching the now exposed adhesive surface etc. until the entire adhesive surface is in contact with the skin. In this way the risk of wrinkles and folds of the dressing sheet during application is minimized and it is easy to position the dressing sheet correctly.

The protection layer may in one embodiment comprise a central portion and a peripheral portion. The central portion may be removed first and the dressing sheet applied to the skin and subsequently the peripheral portion is removed.

The central portion may comprise two or more sections. These sections can be removed one by one before or during application of the dressing sheet to the skin.

The peripheral portion may comprise two or more sections facilitating stepwise detachment of the peripheral portion.

The protection layer may be provided with markings/touch point indicating where to grab the portions for detachment. In one embodiment, the portions may be provided with tab member(s) in the form of an ear or the like.

In one embodiment, the protection layer comprises three portions. In such embodiment one portion is removed and the dressing sheet is applied by holding on the two remaining portions of the protection layer (also known as non-touch).

If the dressing sheet is not positioned as desired, it may be possible to adjust the placement before removing the remaining portion of the protection layer.

The protection layer may have an area substantially being the same size as the adhesive surface of the dressing sheet or it may be larger. Being larger, at least a part of the protection layer may extend further than the edge portion of the dressing sheet, for example in the form of a tab member or in the form of a flange along at least a part of the periphery of the dressing sheet. In one embodiment, the protection layer defines an extended flange along the entire periphery of the dressing sheet.

The wafer may be suitable for a number of medical purposes. The wafer may be a part of an ostomy appliance or the wafer may be a wound dressing.

The wafer may further be provided with an absorbent layer. The absorbent layer may have an area being smaller than the dressing sheet and it may be located at the central portion of the wafer. The absorbent layer may be located on the skin-facing surface of the adhesive layer, optionally being in direct contact with the skin during use or it may be located between the adhesive layer and the backing layer.

The invention relates further to a method of applying an adhesive dressing sheet comprising a backing layer and an adhesive layer to a skin surface comprising the steps of: providing an adhesive wafer comprising an adhesive dressing sheet with a backing layer and an adhesive layer and a detachable porous protection layer covering the skin-facing surface of the adhesive layer, the protection layer comprising a central portion and a peripheral portion, removing the central portion of the protection layer and attaching the thereby exposed adhesive surface of the dressing sheet to the skin and then removing the peripheral portion and attaching the peripheral portion of the dressing sheet to the skin.

The protection layer may work as a reinforcing element during application and handling of the wafer. On conventional wafers the reinforcing elements are often located on the backing layer, not the adhesive layer.

Prior to application to the skin, the protection layer covers the skin contacting side of the adhesive wafer, in order to ensure that the properties of the adhesive are preserved and that the adhesive surface is not laid open until just before the use.

It should be understood that the above description is an exemplary embodiment and that many modifications and alternative embodiments may be provided within the scope of the invention.

### Detailed description of the drawings

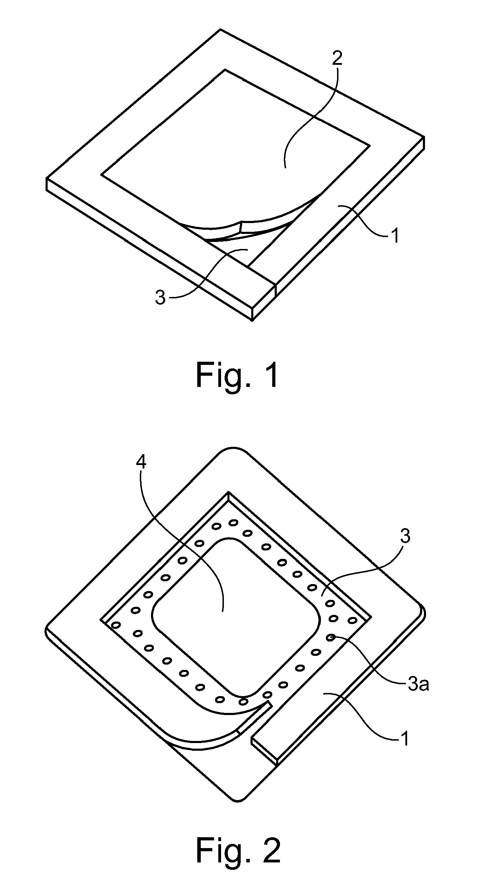

Figure 1 shows an embodiment of the invention shown from the skin facing side. The skin facing surface of the wafer (3) is covered by a protection layer, the protection layer comprising a central portion (2) and a peripheral portion (1). The central portion is about to be detached by the lifted corner exposing the adhesive surface (3) beyond.

Figure 2 shows the same embodiment where the central portion (2) of the protection layer is removed, exposing the central portion of the skin facing surface of the wafer (3). The adhesive layer of the wafer may be pattern coated, comprising zones (3a) without adhesive coating. The wafer may be provided with an absorbent pad (4). The dressing sheet is now applied to the skin, by contacting the adhesive surface of the central portion of the wafer, and then the peripheral portion of the protection layer is removed. By skin facing side/surface is meant the surface facing towards the skin when the dressing sheet is applied.

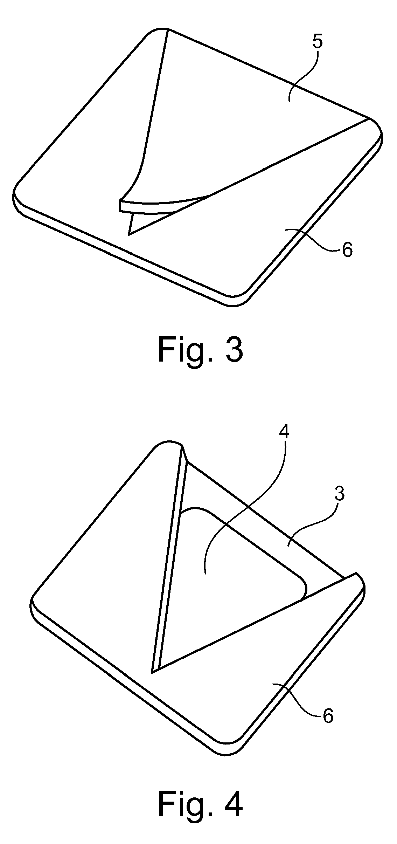

In Figure 3 is shown another embodiment of the invention shown from the skin facing side, where the central portion (5) of the protection layer is triangular. Figure 4 shows the same embodiment where the central portion (5) of the protection layer is removed revealing an absorbent pad (4) and the adhesive skin facing surface (3), and the dressing sheet may be applied to the skin and subsequently the second portion (6) of the protection layer is removed.

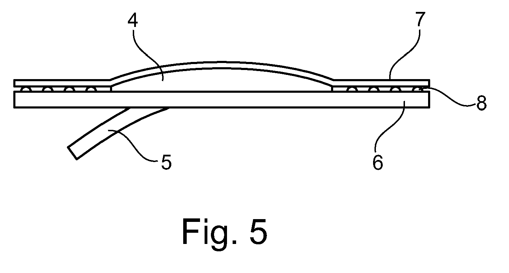

In Figure 5 is shown the embodiment of Figures 3 and 4 seen in cross-section. The wafer comprise a backing layer (7) provided with a pattern coated adhesive layer (8) at the peripheral portion and a central absorbent pad (4). The skin facing surface of the dressing sheet is provided with a porous protection layer (5, 6), being thicker than the adhesive wafer (7,8,9). Detachment of the central portion of the porous protection layer (5) is initiated.

## Claims

1. Adhesive wafer suitable for attachment to the skin or a wound, the wafer comprising
- a dressing sheet comprising a layer of skin-friendly adhesive, the adhesive layer being provided with a backing layer on the non-skin-facing surface thereof,
- and a releasable flexible protection layer on the skin-facing surface of the adhesive layer, the protection layer protecting the adhesive layer before use of the wafer and the protection layer being attached to the adhesive layer in such a way that it is releasable and wherein the protection layer is in the form of a porous material being a foam and having a thickness of 500 µm - 3 mm.

2. Wafer according to claim 1, wherein the foam is an open celled foam.

3. Wafer according to claim 1, wherein the foam is a closed celled foam.

4. Wafer according to any of claims 1-3, wherein the foam is a polyolefine such as polyethylene (PE) or polypropylene (PP) or mixtures thereof.

5. Wafer according to any of the preceding claims, wherein the protection layer has a thickness of 700 µm - 2,5 mm.

6. Wafer according to any of the preceding claims, wherein the protection layer has a thickness of 1-2 mm.

7. Wafer according to any of the preceding claims, wherein the protection layer comprises at least two separately removable portions.

8. Wafer according to any of the preceding claims, wherein the protection layer comprises a central portion and a peripheral portion.

9. Wafer according to claim 8, wherein the central portion comprises two or more sections.

10. Wafer according to claims 8 or 9, wherein the peripheral portion comprises two or more sections.

11. Wafer according to any of the preceding claims, wherein the wafer is provided with a collection bag.

12. Wafer according to any of claims 1-11, wherein the wafer is an ostomy appliance.

13. Wafer according to any of claims 1-10, wherein the wafer is a wound dressing.

14. Method of applying a dressing sheet comprising a backing layer and an adhesive layer to a skin surface comprising the steps of:
- providing an adhesive wafer comprising an adhesive dressing sheet with a backing layer and an adhesive layer and a detachable porous protection layer in the form of a foam having a thickness of 500 µm - 3 mm covering the skin-facing surface of the adhesive layer, the protection layer comprising a central portion and a peripheral portion,
- removing the central portion of the protection layer and attaching the thereby exposed adhesive surface of the dressing sheet to the skin
- and then removing the peripheral portion and
- attaching the peripheral portion of the dressing sheet to the skin.

## Patentansprüche

1. Klebebasisplatte, die für das Anbringen an der Haut oder einer Wunde geeignet ist, umfassend:
- eine Verbandlage, die eine Schicht aus hautfreundlichem Klebstoff umfasst, wobei die Klebstoffschicht an ihrer der Haut nicht zugewandten Fläche mit einer Trägerschicht versehen ist,
- und eine freigebbare flexible Schutzschicht an der der Haut zugewandten Fläche der Klebstoffschicht, wobei die Schutzschicht die Klebstoffschicht schützt, bevor die Basisplatte verwendet wird, und wobei die Schutzschicht so an der Klebstoffschicht angebracht ist, dass sie freigebbar ist, und wobei die Schutzschicht in der Form eines porösen Materials vorliegt, bei dem es sich um einen Schaumstoff handelt und das eine Dicke von 500 µm - 3 mm hat.

2. Basisplatte nach Anspruch 1, wobei der Schaumstoff ein offenzelliger Schaumstoff ist.

3. Basisplatte nach Anspruch 1, wobei der Schaumstoff ein geschlossenzelliger Schaumstoff ist.

4. Basisplatte nach einem der Ansprüche 1 - 3, wobei der Schaumstoff ein Polyolefin wie Polyethylen (PE) oder Polypropylen (PP) oder Mischungen davon ist.

5. Basisplatte nach einem der vorhergehenden Ansprüche, wobei die Schutzschicht eine Dicke von 700 µm - 2,5 mm hat.

6. Basisplatte nach einem der vorhergehenden Ansprüche, wobei die Schutzschicht eine Dicke von von 1 - 2 mm hat.

7. Basisplatte nach einem der vorhergehenden Ansprüche, wobei die Schutzschicht mindestens zwei getrennt entfernbare Abschnitte umfasst.

8. Basisplatte nach einem der vorhergehenden Ansprüche, wobei die Schutzschicht einen mittleren Abschnitt und einen Umfangsabschnitt hat.

9. Basisplatte nach Anspruch 8, wobei der mittlere Abschnitt zwei oder mehr Sektionen umfasst.

10. Basisplatte nach Anspruch 8 oder 9, wobei der Umfangsabschnitt zwei oder mehr Sektionen umfasst.

11. Basisplatte nach einem der vorhergehenden Ansprüche, wobei die Basisplatte mit einem Sammelbeutel versehen ist.

12. Basisplatte nach einem der Ansprüche 1 - 11, wobei die Basisplatte eine Stomavorrichtung ist.

13. Basisplatte nach einem der Ansprüche 1 - 10, wobei die Basisplatte ein Wundverband ist.

14. Verfahren zum Aufbringen einer Verbandlage, die eine Trägerschicht und eine Klebstoffschicht umfasst, auf eine Hautfläche, das die folgenden Schritte umfasst:
- Bereitstellen einer Klebebasisplatte, die eine Klebeverbandlage mit einer Trägerschicht und einer Klebstoffschicht und einer ablösbaren porösen Schutzschicht in der Form eines Schaumstoffs mit einer Dicke von 500 µm - 3 mm umfasst, die die der Haut zugewandte Fläche der Klebstoffschicht abdeckt, wobei die Schutzschicht einen mittleren Abschnitt und einen Umfangsabschnitt umfasst,
- Entfernen des mittleren Abschnitts der Schutzschicht und Anbringen der dadurch freigelegten Klebstofffläche der Verbandlage an der Haut
- und dann Entfernen des Umfangsabschnitts und
- Anbringen des Umfangsabschnitts der Verbandlage an der Haut.

## Revendications

1. Plaquette adhésive appropriée pour être fixée sur la peau ou une blessure, la plaquette comprenant :
- un pansement comprenant une couche d'adhésif dermocompatible, la couche d'adhésif étant pourvue d'une couche dorsale sur la surface du côté opposé à la peau, et
- une couche de protection souple amovible sur la surface côté peau de la couche d'adhésif, la couche de protection protégeant la couche d'adhésif avant utilisation de la plaquette et la couche de protection étant fixée à la couche d'adhésif de façon à être amovible, dans laquelle la couche de protection se présente sous la forme d'un matière poreuse qui est une mousse et a une épaisseur de 500 µm à 3 mm.

2. Plaquette selon la revendication 1, dans laquelle la mousse est une mousse à alvéoles ouverts.

3. Plaquette selon la revendication 1, dans laquelle la mousse est une mousse à alvéoles fermés.

4. Plaquette selon l'une quelconque des revendications 1 à 3, dans laquelle la mousse est une polyoléfine telle que le polyéthylène (PE) ou le polypropylène (PP) ou un mélange de ces produits.

5. Plaquette selon l'une quelconque des revendications précédentes, dans laquelle la couche de protection a une épaisseur de 700 µm à 2,5 mm.

6. Plaquette selon l'une quelconque des revendications précédentes, dans laquelle la couche de protection a une épaisseur de 1 à 2 mm.

7. Plaquette selon l'une quelconque des revendications précédentes, dans laquelle la couche de protection comprend au moins deux parties amovibles séparément.

8. Plaquette selon l'une quelconque des revendications précédentes, dans laquelle la couche de protection comprend une partie centrale et une partie périphérique.

9. Plaquette selon la revendication 8, dans laquelle la partie centrale comprend deux ou plusieurs sections.

10. Plaquette selon la revendication 8 ou 9, dans laquelle la partie périphérique comprend deux ou plusieurs sections.

11. Plaquette selon l'une quelconque des revendications précédentes, dans laquelle la plaquette est pourvue d'un sac collecteur.

12. Plaquette selon l'une quelconque des revendications 1 à 11, dans laquelle la plaquette est un appareillage stomique.

13. Plaquette selon l'une quelconque des revendications 1 à 10, dans laquelle la plaquette est un pansement pour plaie.

14. Procédé d'application sur une surface de peau d'un pansement qui comprend une couche dorsale et une couche d'adhésif, comprenant les étapes consistant à :
- fournir une plaquette adhésive, comprenant un pansement adhésif, ayant une couche dorsale et une couche d'adhésif, et une couche de protection poreuse amovible se présentant sous la forme d'une mousse ayant une épaisseur de 500 µm à 3 mm qui recouvre la surface côté peau de la couche d'adhésif, la couche de protection comprenant une partie centrale et une partie périphérique,
- retirer la partie centrale de la couche de protection et fixer sur la peau la surface adhésive ainsi à découvert du pansement, puis
- retirer la partie périphérique, et
- fixer sur la peau la partie périphérique du pansement.
